# EUROPEAN PATENT SPECIFICATION

(11) **EP 2 990 024 B1**
(45) Date of publication and mention of the grant of the patent: **25.09.2019**
(21) Application number: 14182224.7
(22) Date of filing: 26.08.2014
(51) Int. Cl.: A61K 8/00, C09D 11/02, C09D 11/03, C09D 11/322

(54) **Pigment concentrate**
Pigmentkonzentrat
Concentré de pigment

(43) Date of publication of application: 02.03.2016
(73) Proprietor: Oleon N.V., 9940 Ertvelde (BE)
(72) Inventor: Packet, Dirk, 3110 Rotselaar (BE); Zitouni, Karima, 91420 Morangis (FR); Huybrechts, Ward, 2600 Berchem (BE); Nees, Wilfried, 2160 Wommelgem (BE)
(74) Representative: V.O.

(56) References cited:
- WO-A1-01/83488
- WO-A1-99/45060
- WO-A1-2013/017262
- DE-A1- 19 723 732
- JP-A- S59 175 408
- JP-A- 2014 172 870
- US-A1- 2011 315 049
- ANONYMOUS: "High boiling organic solvents for dispersion of photographic additives", RESEARCH DISCLOSURE, MASON PUBLICATIONS, HAMPSHIRE, GB, vol. 158, no. 55, 1 June 1977 (1977-06-01) , XP007104870, ISSN: 0374-4353

## Description

The present invention relates to a pigment concentrate comprising particles of one or more pigments dispersed in at least one dispersing agent.

Such pigment concentrates find wide application in printing ink compositions, in particular in offset printing compositions.

Printing inks find application in a wide variety of printing processes. Frequently used printing processes include photogravure, flexography, rotogravure, and last but not least offset lithography printing, which has become a frequently used printing method. For each type of printing process, dedicated printing ink formulations have been developed with a composition balanced to meet the requirements of the printing process and the end use properties. Lithographic printing traditionally uses a two-component ink system comprising a hydrophilic fountain solution in combination with a hydrophobic ink paste.

Printing inks are usually manufactured by combining a pigment with a vehicle or carrier, one or more additives and liquids, to form a dispersion with a desired colouring power and printability. More economic manufacturing processes make use of a pigment concentrate, which has a noticeably higher pigment concentration than the printing ink. Pigment concentrates used to prepare inks are typically provided in the form of slurries based on water or a non-aqueous dispersing agent. The printing ink may be produced by dilution mixing of the pigment concentrate with a vehicle and liquids such as oils and any other additives required. The dispersion step to obtain a dispersion of pigment particles of a desired size in the dispersing agent can be dispensed with, since the pigment particles are already present in a dispersed state in the pigment concentrate. Pigment concentrates may also be used to increase the concentration of the colouring material of a printing ink of the same shade.

The pigment appears to be the most costly component of an ink composition. In view hereof, many industrial activities concentrated on maximizing colour strength of the pigmented inks, image gloss and colour density while minimizing the amount of pigment used in each batch of ink. Various attempts sought to improve upon these properties, by providing pigment particles with a smaller particle size, by modifying the surface properties of the pigment particles to prevent agglomeration, or by producing inks with stable pigment dispersions with a low settling or agglomeration rate of pigment particles. It is generally assumed that small pigment particles provide better colour strength, saturation, gloss, hiding power, dispersion and flow. Large particles tend to exhibit poor dispersion causing fast cylinder wear, poor ink/water balance, poor flow, poor hiding power, poor colour strength and colour fluctuation, and lower ink gloss.

The quality of a printed image strongly depends on the colour strength and opacity of the ink used. With colour strength is meant the effective concentration of colouring materials per unit weight or volume of ink. Colour strength tends to increase with decreasing particle size, because of the larger surface area per unit mass and better bonding among the particles. Colour strength has been found to also depend on factors such as pigment particle size distribution, particle shape, and degree of dispersion of the pigment particles. Colour strength may be determined qualitatively or quantitatively according to the CIELAB system of the Commission Internationale de l'Eclairage, described in Kirk-Othmer Encyclopedia of Chemical Technology 6:523-548 (1979). L* represents the lightness of the ink with small values down to zero indicating "darkness" and large value up to 100 indicating "brightness." a* represents red or green, with more positive values indicating "redness" and more negative values indicating "greenness." b* represents yellow or blue, with more positive values indicating "yellowness" and more negative values indicating "blueness."

With opacity is meant the ability of a pigment to cover or obscure the surface to which it is applied. Varying degrees of opacity/transparency may be obtained by varying the nature of the pigment and its dispersion degree in the ink formulation. Small pigment particles tend to build inks with a higher transparency, opacity of an ink pigment reaches a maximum around 200-550 nm.

Gloss may be defined as the percentage of light reflected at the same angle as the angle of incidence. The typically used total angle is 60° (Kirk-Othmer Encyclopedia of Chemical Technology 16:745 (1981), describing gloss in the context of paint). Colour density is a strength of colour which is categorized by black and white, blue (or cyan), red (or magenta) or yellow. A densitometer may be used to measure the strength of darkness, blueness, redness and yellowness in a given ink sample. The colour strength, gloss, and density of a pigmented ink may be generally referred to as the colour properties of the ink.

US5.919.838 discloses a method for preparing ink concentrates from aqueous slurries of pigment particles or from dry pigment particles for example a press cake, wherein the pigment particles are blended with a varnish including a hydrophobic carrier, a supporting resin, a wetting polymer and at least one dispersing polymer. The blend is heated to melt the dispersing polymer. Upon cooling, the polymer solidifies on the surface of the pigment particles to prevent the particles from agglomerating. Inks may be prepared from the ink concentrates thus provided. The ink is said to have superior colour strength and gloss. The method disclosed in US5.919.838 comprises the steps of :
(a) adding a varnish to an aqueous slurry of pigment particles to thereby form a mixture of varnish and slurry, the varnish including at least one dispersing polymer,
(b) removing water from the mixture to leave a blend of varnish and pigment;
(c) heating the blend to a temperature effective to melt the dispersing polymer and to drive off any remaining water in the blend; and
(d) cooling the blend to thereby form an ink concentrate.

In view of the fact that the pigment is the most costly component of the ink composition, a need still exists to improve the colour properties of pigmented inks to provide superior colour strength, gloss and colour density. In addition hereto, an increasing need exists to produce industrial products using renewable materials.

### SUMMARY OF THE INVENTION

The present invention therefore seeks to provide a pigment concentrate which is suitable for use in offset printing inks and the like, which may be made of renewable raw materials in an economically feasible manner.

This is achieved according to the present invention by using as a dispersing agent for the preparation of a pigment concentrate comprising particles of one or more pigments a monomeric ester of a polyol with at least one carboxylic according to formula I 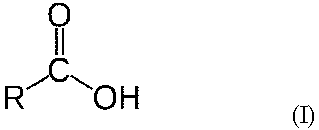 wherein R is a hydrocarbon rest containing from 3 up to 27 carbon atoms, wherein the polyol comprises isosorbide and the average degree of esterification of the polyol moieties in the isosorbide ester ranges from 1.50 to 1.99, more preferably from 1.60 to 1.98, in particular from 1.70 to 1.97, more in particular from 1.75 to 1.95 and wherein the monomeric ester is a mixture comprising monoesters and polyesters of isosorbide, which mixture comprises isosorbide monoester at a level of at most 50 wt% based on the total weight of the ester mixture, preferably at most 30 wt% monoester, more preferably at most 20 wt% and even more preferably at most 10 wt% monoester.

The inventors have found that the isosorbide ester according to the present invention combines very desirable properties for its particular use as a dispersion agent in a pigment concentrate, and that it is capable of transferring these desirable properties to the pigment concentrate containing it and further towards a composition containing it, for example a printing ink composition containing the pigment concentrate, in particular offset printing ink compositions, a cosmetic composition for example a white or coloured cream, etc.

The inventors have particularly found that the isosorbide ester of this invention is capable of offering a desirably high wetting power for wetting the pigment particles in the pigment concentrate. This high wetting power for the pigment particles is very desirable, as it counteracts and limits re-agglomeration of pigment particles after they have been pulverized to a desired size to prepare a desired dispersion, and supports and stabilises the pigment particles in a dispersed form in a desired particle size or particle size distribution. The high wetting power permits to maintain the particle size of the pigment particles at a desirable particle size distribution for a longer period of time, thereby improving the shelf life of a formulation. The inventors have observed that the use of the ester of the present invention permits to provide a pigment dispersion with an improved stability over a reasonable range of time and temperature, even at temperatures up to 50°C or more and when subjecting a pigment concentrate to freeze/thaw cycles. Temperatures of 50°C or more typically prevail in the inking unit of offset printers. The stability of the pigment particle dispersion in the pigment concentrate has been observed even with small particles.

The stabilizing effect offered by the isosorbide ester brings many advantages not only to the pigment concentrate, but also to a composition, for example an offset printing ink composition or a cosmetic composition, containing the pigment concentrate of this invention. The stable dispersion in the pigment concentrate, supports a homogeneous distribution of the pigment in a composition comprising such a pigment concentrate.

Thus, with the dispersion agent of the present invention, a pigment concentrate may be obtained which is capable of providing improved colour properties, in particular improved colour strength, gloss, density and opacity.

The inventors have further found that the pigment concentrate of this invention offers excellent performance in terms of spreadability on various substrates, homogeneity and colour intensity or density of an image printed on a substrate.

The inventors have also found that the isosorbide ester according to the present invention may bring the advantage of being non-toxic and may have a low odour and/or taste, such that a pigment concentrate containing the ester may be suitable for use in more sensitive applications, for example on substrates which come into contact with foodstuffs.

Without wanting to be bound by this theory, the inventors assume that the combination of the highly desirable properties described above may be at least partially be implied by the molecular structure of the isosorbide esters, which may at least partially be brought by the bulkiness of the furan rings.

The properties of the pigment concentrate, in particular the colour strength, the dispersion degree and stability of the dispersion, may be tuned by an appropriate selection of the carboxylic acid or carboxylic acid mixture used for preparing the ester, and by varying the nature and/or composition of the carboxylic acid moiety of the ester. The applicants have found that these properties and in particular the balance of properties, may be further tuned by adaptation of the ester, for instance by varying its degree of esterification, by varying the nature of the carboxylic acid and/or the presence of minor other polyol moieties, such as sorbitan in the isosorbide raw material. Such tuning may be desirable to take into account varying nature of the pigments.

The isosorbide ester according to the present invention may be produced almost exclusively from renewable raw materials. Whereas the isosorbide polyol part of the ester is preferably obtained from glucose or dextrose, which may be obtained from starch, the carboxylic acid part of the isosorbide ester may for instance be a fatty acid or a mixture of several fatty acids obtained from fats which originate from vegetal products such as for example corn, rapeseed, coconut, cuphea, oil palm and the fruit kernels thereof, and many others although synthetic carboxylic acids may be used as well.

It is remarked that a pigment concentrate differs from for example an ink composition in its generally higher pigment concentration and the limited number of pigment types contained in it - usually one, sometimes two. A pigment concentrate generally distinguishes itself by a higher pigment loads than colour strength of the final application requires.

In a preferred embodiment of this invention, the at least one dispersing agent mainly consists of one or more esters of isosorbide with at least one C₆-C₂₆ fatty acid, i.e. fatty acids in which R contains between 5 and 25 carbon atoms.

### DETAILED DESCRIPTION.

The present invention will be described in more detail in the part following hereafter, with respect to particular embodiments but the invention is not limited thereto.

Isosorbide (CAS No 652-67-5) is a bicyclic diol, composed of two adjacent and saturated furan rings, and which corresponds to formula II below:

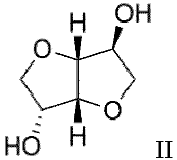

The isosorbide molecule has a rigid and chiral structure, shows good heat stability and is non-toxic. Isosorbide is considered a renewable material which may be produced from biomass, from a.o. glucose or starch. Isosorbide is typically obtained by the hydrogenation of the aldehyde function in glucose (a.k.a dextrose) to the corresponding alcohol function, thereby producing sorbitol. Sorbitol has a structure based on a straight chain carbon backbone having six carbon atoms, each of them carrying one hydroxyl function. Sorbitol may be represented by the formula III: 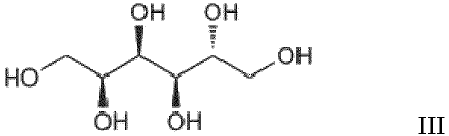 Single dehydration of sorbitol produces sorbitan, comprising one saturated furan ring and having still four hydroxyl functions. Sorbitan may be represented by the formula IV: 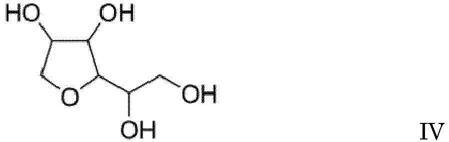 Double dehydration of sorbitol produces the isosorbide, having only two hydroxyl functions left.

Esters of isosorbide are known in the art.

WO 2013/017261 A1 discloses compositions containing isosorbide monoesters and isosorbide diesters, prepared by esterification of isosorbide, commercially obtained as "Sorbon" from Ecogreen Oleochemicals, with caprylic (octanoic) acid. The monoester product contains 51 wt.% of the monocaprylate, 31 wt.% of the dicaprylate and 18 wt.% remaining isosorbide. The diester product contains 90 wt.% of the dicaprylate and 9% of the monocaprylate. Both products are shown to be effective thickeners for surfactants when added at 1 wt.% to the composition. The monoester product is further shown to exhibit antimicrobial activity, by controlling the growth of a number of bacteria, fungi and yeasts, at concentration levels below 10000 ppm wt. Primarily the monoester product is then proposed for use in the preparation of a cosmetic, dermatologic or pharmaceutic composition, a plant protection formulation, a wash- or cleaning agent or a colour or coating agent.

WO 2013/017257 A1 describes the same monoester and diester products as in WO 2013/017261 A1, and demonstrates that it is only the monocaprylate which is responsible for the antimicrobial activity observed, and that isosorbide itself, nor the dicaprylate, exhibit any such activity. Again, the monoester product is proposed for use in the preparation of a cosmetic, dermatologic or pharmaceutic composition, a plant protection formulation, a wash- or cleaning agent or a colour or coating agent. WO 2013/017255 A1 describes the same monoester and diester products, and demonstrates the effect of isosorbide monocaprylate as a thickener of an aqueous surfactant composition, at levels of up to 2.0 wt.%. WO 2013/017256 A1 describes the same diester product, and demonstrates the effect thereof as a thickener of an aqueous surfactant composition, at a level of 1.0 wt.%. In both latter documents, the use of their isosorbide esters are proposed in the preparation of a cosmetic, dermatologic or pharmaceutic composition, a plant protection formulation, a wash- or cleaning agent or a colour or coating agent.

WO 99/45060 describes the use of isosorbide esters as plasticizer for polymer materials. Diesters are mentioned but without providing any information as to potential co-presence of monoesters.

US 2011/315049 relates to an inkjet composition containing a pigment, a pigment dispersant and a solvent composition. The solvent can be isosorbide dicaprylate; nothing is mentioned about potential co-presence of monoesters.

The article titled "High boiling organic solvents for dispersion of photographic additives" published in Research Disclosure, Vol 158, No 55 of 1977, refers to the use of isosorbide dicaprylate for dispersing photographic additives. No information is provided on potential co-presence of monoesters.

WO 2013/017262 discloses antibacterial compositions comprising a mono ester of isosorbide and a halogenated antimicrobially active compound having an advantageous preservation performance and stability to microbial attack. Diester of isosorbide can be present in an amount of 9 to 50 wt% on the ester mixture.. There is no reference to a potential pigment dispersing activity of the isosorbide ester. Further the concentration of pigment, if present, is at most 20 wt%.

DE 19723732 describes the use of monoacylesters of isosorbide such as isosorbide monolaurate as solubilizer for light stabilizers in cosmetic preparations. There's no indication of any polyester being present.

JP 2014/172870 and JP 59/175408 relate to cosmetic compositions containing isosorbide difatty acid ester as an oily component to provide excellent diffusibility and feeling to the skin. There is no mentioning of presence of monoesters of isosorbide.

WO 01/83488 discloses a production method for diesters of isosorbide.

These documents however fail to disclose the excellence which isosorbide esters may exhibit as dispersion agent in the formulation of pigment concentrates, and in the further formulation of such pigment concentrates to printing inks or varnishes and associated compositions.

Within the scope of this invention either one or both of the OH groups of the isosorbide may be esterified with a carboxylic acid. Also within the scope of this invention, the isosorbide ester is a mixture of monoesters and diesters of isosorbide with at least one carboxylic acid. The degree of esterification of the isosorbide may vary within a range of between 1.50-1.99, more preferably in the range of 1.60-1.98, even more preferably in the range of 1.70-1.97, and yet more preferably in the range of 1.75 -1.95. The degree of esterification of the isosorbide may affect its properties, and therefore within the scope of this invention the degree of esterification of the isosorbide ester may be selected by the skilled person taking into account the nature of the ester and/or other components present in the pigment concentrate and if so desired, the nature of the surface which is to be printed.

The amount of free, non-esterified isosorbide contained in the isosorbide ester composition is preferably as low as possible as free isosorbide may be reactive with other components present in the pigment concentrate, or the printing ink composition into which the pigment concentrate it is to be incorporated. The presence of non-reacted isosorbide may thereby adversely affect the viscosity of the pigment concentrate.

The isosorbide ester used in the pigment concentrate of the present invention has been found to be characterised by a relatively high kauri-butanol (Kb) value, which will usually be at least 25, preferably at least 50, more preferably at least 60, even more preferably at least 70, yet more preferably at least 80. Although there is no need to set an upper limit, the Kb value of the isosorbide ester of this invention will usually not be more than 250, often at most 200 or 170, preferably at most 160, more preferably at most 150 and even more preferably at most 140. Therewith the isosorbide ester imparts to the pigment concentrate a good compatibility with a wide variety of printing ink and printing varnish compositions in which the pigment concentrate is to be incorporated, and may contribute to dissolving resin material present therein. The "Kb value" is a measure of solvent power, and may be determined using the standardized test ASTM D1133. A high Kb value is an indication that the isosorbide ester is more capable in dissolving a wide range of resins of varying nature, and in higher concentrations, as compared to solvents having a lower Kb value. Without wanting to be bound by this theory, the applicants believe that this advantageous combination of properties, i.e. high wetting power and high Kb value, must be attributed to the structure of the isosorbide moiety in the ester molecule. The fact that the isosorbide ester is capable of functioning as a solvent for other components present in an ink composition into which the pigment concentrate is to be incorporated is important as offset inks usually contain besides one or more pigments, approximately 40-65 wt. % of resin binders, for example rosin resins, maleic resins, hydrocarbon resins and alkyd resins, to ensure formation of a polymer film which binds the pigments or dyes together and provides resistance properties. Such resin binders are usually absent in inkjet printing inks. The offset printing ink may further contain additives, for example waxes, siccative agents and/or inhibitors. Such additives will usually be present in a concentration of 3 - 6 wt. %.

The isosorbide polyol raw material may contain traces of sorbitol as an impurity. Sorbitol is a polyol having six OH functions as an impurity. The content of free, non esterified sorbitol in the ester is preferably as low as possible as it may be reactive with other components present in a composition, in particular the pigment concentrate, and may thereby adversely affect the viscosity of the pigment concentrate and even the viscosity of an offset printing ink composition or an offset printing varnish composition containing the pigment concentrate of this invention. When sorbitol is present, it is within the scope of the present invention that one, two, three, four, five or all of the OH groups of sorbitol may be esterified with a carboxylic acid. Within the present invention therefore, the dispersing agent composition according to the present invention may contain one or more sorbitol esters, and may contain a mixture of sorbitol monoesters, di-esters, tri-esters, tetra-esters, penta-esters and hexa-esters.

The isosorbide polyol raw material may also contain traces of sorbitan as an impurity. When sorbitan is present, it is within the scope of the present invention that one, two, three, or all four of the OH groups of the sorbitan may be esterified with a carboxylic acid. The dispersing agent composition according to the present invention may thus contain one or more sorbitan esters, and may contain a mixture of sorbitan monoesters, di-esters, tri-esters and tetra-esters with at least one carboxylic acid. The content of free, non esterified sorbitan in the ester is preferably as low as possible as it may be reactive with other components present in a composition and may thereby adversely affect the viscosity of an offset printing ink composition or an offset printing varnish composition containing the vehicle of this invention.

It is thus within the scope of this invention that where it is mentioned that polyol comprises or consists of isosorbide, "isosorbide" is meant to include one or more of the above described impurities.

The applicants have found that the degree of esterification of the polyol starting material has a strong effect on the performances of the isosorbide ester. The applicants have therefore defined a "degree of esterification", which is determined by the amount of free OH functions remaining in the ester product and/or the dispersing agent composition.

In an embodiment of the present invention, the ester has a hydroxyl value of at most 50 mg KOH/g, preferably at most 40 mg KOH/g, more preferably at most 30 mg KOH/g and even more preferably at most 20.0 mg KOH/g. The hydroxyl value is to some degree related to the monoester content present in the isosorbide ester. However, due to the usual presence of sorbitan and/or sorbitol side products in the isosorbide polyol raw material, although in minor amounts, these compounds having more than 2 hydroxyl functions per molecule render this relationship more complex and the relationship is influenced by other factors also. The applicants therefore prefer to also control the hydroxyl value of the ester as defined in the context of the present invention, and optionally over and above the monoester content.

In an embodiment of the pigment concentrate of the present invention, the fatty isosorbide ester has a hydroxyl value of at least 0.5 mg KOH/g, preferably at least 2.0 or at least 5.0 mg KOH/g, preferably at least 8.0 mg KOH/g, more preferably at least 10.0 mg KOH/g and even more preferably at least 15.0 mg KOH/g. The applicants have found that the remaining OH functions that have not been esterified, bring the advantage of wetting power and therewith contribute to the stabilisation of the pigment particles in their dispersed state. Over and above controlling the monoester content of the ester composition, the applicants therefore prefer to also control the hydroxyl value of the ester.

Because the pigment particles may be stabilised in their dispersed state a more homogeneous pigment concentrate may be obtained and therewith a more homogeneous pigment distribution in an image printed with a printing ink containing the pigment concentrate of the present invention, as well as a good colour strength without having to increase the pigment concentration, and higher gloss. The inventors have in particular found that an improved colour strength may be obtained at low pigment concentrations when compared to prior art pigment concentrates and ink compositions containing such a pigment concentrate.

In an embodiment of the pigment concentrate according to the present invention, the ester is a mixture which comprises isosorbide monoester at a level of at least 1.0 wt.% based on the total weight of the ester mixture, preferably at least 3.0 wt.% monoester, more preferably at least 4.0 wt.% and even more preferably at least 5.0 wt.% monoester. The isosorbide ester is a mixture which comprises isosorbide monoester at a level of at most 50 wt.% based on the total weight of the ester mixture, preferably at most 30 wt.% monoester, more preferably at most 20 wt.% and even more preferably at most 10 wt.% monoester.

The applicants have found that the mono-ester of isosorbide brings wetting power to the pigment particles contained in the pigment concentrate so that agglomeration may be counteracted. The wetting power is also beneficial for quickly wetting the substrate that is to be printed, thereby improving adhesion of the pigment to the substrate and improving quality, in particular gloss and colour homogeneity of an ink or varnish applied to the substrate surface. The wetting power of the isosorbide ester is also beneficial for quickly wetting other solid particles, such as resin particles contained in the pigment concentrate, in a varnish composition with which the pigment concentrate may be mixed to form an ink composition, or in an ink composition. This may bring advantages in the stability and the shelf life of the pigment concentrate according to the present invention. The applicants have however further found that a too high content of monoester may increase the viscosity of the ester composition which could adversely affect the miscibility of the pigment concentrate with the other components of a printing ink or varnish composition. To prevent that the viscosity of the pigment concentrate would become too high the amount of monoester present is limited to the specified upper limits. The applicants have also observed that the isosorbide mono-ester brings spreadability to the pigment concentrate and to an offset printing ink or varnish offset printing composition containing that pigment concentrate.

The monoester of the isosorbide ester as defined in the present invention may be readily determined by measuring the hydroxyl number, and assuming that only isosorbide monoester and isosorbide diester are present, hence assuming that all the hydroxyl functionality is due to the monoester presence. Unreacted isosorbide will usually only be present in trace amounts as has been described above. Any amount of unreacted isosorbide present may for example be measured using gas chromatography (GC).

In an embodiment of the present invention, the dynamic viscosity at 25°C of the isosorbide ester according to the present invention is preferably in the range of 5-600 mPa.s, preferably at least 20 mPa.s, preferably at least 40 mPa.s, optionally at most 400 mPa.s, preferably at most 300 mPa.s, more preferably at most 200 mPa.s and even more preferably at most 100 mPa.s. This viscosity range provides pigment dispersions with optimal dispersion stability, as well as a good miscibility with other components of an ink composition, in particular an offset ink composition into which the pigment concentrate is to be incorporated and allows a wide degree of freedom in the use of a pigment concentrate of this invention for the formulation of offset printing inks or varnishes having the correct final viscosity required for the application. The inventors have observed that a low viscosity permits achieving good drying properties of the ink, due to good migration ability of the ink. The viscosity is measured according to ASTMD 445.

The carboxylic acids used in the ester of the present invention responds to formula I: 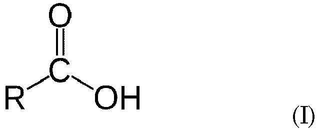 wherein R is a hydrocarbon rest containing from 3 up to 27 carbon atoms, preferably from 3 up to 25 carbon atoms. Varying the length of the hydrocarbon chain permits to tune polarity of the dispersing agent if the pigment, other components of the pigment concentrate or the application in the final ink so require.

In formula I, R may either be a straight chain, branched or cyclic hydrocarbon rest. Thus, the carboxylic acid used to produce the isosorbide ester may either be a straight chain, a branched or cyclic carboxylic acid. Furthermore, R may be saturated or may contain one or more unsaturated bonds, or it may be aromatic. In particular R may contain one or more single or double unsaturated C-C bonds. The carboxylic acid used to produce the isosorbide ester may for example be a straight chain or a branched saturated carboxylic acid, a straight chain or a branched carboxylic acid containing one or more unsaturated bonds, an aromatic carboxylic acid etc. Examples of carboxylic acid suitable for use with the present invention include benzoic acid, 2-ethylhexylic acid, iso-nonanoic acid, octanoic acid, butyric acid, valeric acid etc.

The carboxylic acid used to produce the isosorbide ester may comprise one single type of carboxylic acid, or may be a mixture of two or more different carboxylic acids, depending on the envisaged properties. The carboxylic acids may thereby differ in their composition and/or their chemical structure.

Preferred carboxylic acids are those in which R comprises from 5 up to 17 carbon atoms, preferably from 5 up to 15 carbon atoms.

In preparing the isosorbide ester, carboxylic acids of natural origin may be used as the carboxylic acid, in particular fatty acids of natural origin, although synthetic carboxylic acids may be used as well. When fatty acids are used which originate from natural oils and/or fats, the fatty acid part of the fatty acid ester will usually comprise a mixture of fatty acids with a varying number of carbon atoms, in particular a mixture of C6-C28 fatty acids, i.e. fatty acids in which the R group contains between 5 and 27 carbon atoms. The composition of the fatty acid mixture may vary, and it may vary a.o. with the nature of the fat from which they are derived, and with any pre-treatment to which the oil and/or fat has been subjected, such as for example refining.

The fatty acid mixture may exclusively consist of saturated fatty acids, or it may contain fatty acids containing one or more unsaturated bonds. Unsaturated bonds a.o. include C=C double bonds. The use of saturated acids is preferred over unsaturated fatty acids as they produce less odour as compared to unsaturated fatty acids, and show a better resistance to oxidation and a better heat stability, so that the risk for the formation of unwanted by-products is minimised. The use of saturated fatty acids permits a.o. limiting the risk to so-called yellowing of white inks as well as the risk to the formation of cracks during or after printing.

In an embodiment of the present invention, the fatty acid mixture is a mixture which comprises at least 75 wt.% of C₄-C₁₂ fatty acids, i.e. fatty acids in which the R group comprises between 3 and 11 carbon atoms, preferably C₆-C₁₂ fatty acids, i.e. fatty acids in which the R group comprises preferably between 5 and 11 carbon atoms. More preferably the content of such fatty acids is at least 85 wt.%, even more preferably at least 90.0 wt.%, yet more preferably at least 95.0 wt.%. The applicants have found that these shorter chain fatty acids bring the advantage that the pour point of the ester is reduced. The lower carbon number fatty acids provide esters which are more fluid, and remain fluid at lower temperatures, which is an advantage in the use of such esters as dispersing agent in the preparation of pigment concentrates and their subsequent formulation as a printing ink, in particular an offset printing ink. Preferably the melting point and the pour point of the isosorbide ester is below room temperature (23°C), preferably at most 20°C, more preferably at most 10°C, even more preferably at most 0°C. A further advantage of such low melting point is that the final composition remains clear at room temperature, and preferably also the intermediates thereof.

In an embodiment of the present invention, the fatty acid mixture is a mixture in which the content of fatty acids having between 12 and 22 carbon atoms is preferably at most 15 wt.%, more preferably at most 10.0 wt.%, even more preferably at most 5.0 wt.%, yet more preferably at most 3.0 wt.%. The applicants have found that this feature brings the advantage or keeping the pour point of the ester and of the dispersing agent composition low, and transfers this advantage to the compositions containing them.

In an embodiment of the present invention, the fatty acid is a mixture of fatty acids of which the content of C₁₂-C₂₂ fatty acids, i.e. fatty acids in which the R group contains between 11 and 17 carbon atoms is at most 5.0 wt.%, preferably at most 3.0 wt.%. The applicants have found that this helps in keeping the pour point low, as explained in the preceding paragraph, in particular if the mixture is rich in saturated fatty acids.

Suitable examples of saturated straight chain C₆-C₁₂ fatty acids include hexanoic acid (caproic acid), heptanoic acid (enanthic acid), octanoic acid (caprylic acid), nonanoic acid (pelargonic acid), decanoic acid (capric acid), undecanoic acid, dodecanoic acid (lauric acid), and examples of unsaturated straight chain fatty acids may comprise undecenoic acid and lauroleic acid, and mixtures of two or more thereof. Suitable saturated fatty acids having higher carbon numbers include tetra-decanoic acid (myristic acid), hexa-decanoic acid (palmitic acid), octa-decanoic acid (stearic acid), eicosanoic acid (arachnidan acid), and docosanoic acid (behenic acid).

Suitable examples of unsaturated fatty acids include tetra-decenoic acid (myristoleic acid), hexa-decenoic acid (palmitoleic acid and/or sapeinic acid), octa-decenoic acid (oleic acid, or other isomers depending on the location of the double bond), octa-decadienic acid (linoleic acid, in its various forms depending on the location of the double bonds), and alpha-linolenic acid (C18:3).

Suitable examples of synthetic carboxylic acids include iso-nonanoic acid, 2-ethylhexylic acid, benzoic acid, branched carboxylic acids, for example cekanoic acids, neo acids etc. For the synthesis of branched carboxylic acids the following industrial production processes are particularly mentioned. Each of these processes is considered suitable for producing branched acids suitable for use within the present invention, in particular for producing cekanoic acids. Examples of cekanoic acids suitable for use with the present invention are C₈ cekanoic acid or iso-octanoic acid, and C₁₀ cekanoic acid. A first process for producing such acids comprises carboxylation of olefins in the so-called Koch process. According to this process carbon mono oxide and water addition to olefins leads directly to carboxylic acids. Depending on the nature of the olefin, either cekanoic acids or neo acids may be obtained. According to another process carbonylation of olefins gives aldehydes, which may be oxidized to carboxylic acids. Similar acids can be made as with the Koch process. According to a further process use is made of oxidation of aldehydes, obtained by aldol condensation. This type of process may be used for the production of for example 2-ethylhexanoic acid.

A class of carboxylic acids that are considered to be particularly useful for the present invention include neo acids. Neo acids are highly branched synthetic tri-alkyl carboxylic acids in which the carboxylic group is attached to a quaternary carbon atom, where the other three substituents are alkyl groups. Neo acids may be manufactured by reacting an olefin with a high purity carbon monoxide under high pressure. Examples of commercially available neoacids suitable for use with this invention include those having from 5 up to 28 carbon atoms in the branched carbon chain, for example neo-pentanoic acid or 2,2-dimethylpropanoic acid, neo-heptaonoic acid or 4,4-dimethylpentanoic acid, neo-decanoic acid or 2,2-dimethyloctanoic acid, C₁₃ neoacid, C₈ neo acid or 2-ethylhexanoic acid. Other neo acids include neo fatty acids, in particular fatty acids having a terminal tertiary butyl group or two iso-methyl groups, for example 13,13-diemthyltetradecanoic acid or neo-palmitic acid, and C₉-C₁₃ neo fatty acids and C₉-C₂₈ neo fatty acids.

The isosorbide ester of the present invention may be prepared by a reaction of isosorbide with a carboxylic acid, in particular a mixture of two or more carboxylic acids or a mixture of two or more fatty acids, having a varying number of carbon atoms. Thereby preferably a carboxylic acid mixture or a fatty acid mixture may be chosen such that the ester as defined in the invention is liquid at the temperature at which the printing ink is to be used. This may be achieved by selecting a carboxylic acid of which the carbon number, or a mixture of carboxylic acids of which the weight average carbon number, is in the preferred ranges mentioned above, such as a mixture of C₆-C₁₂ fatty acids. Particularly preferred are the fatty acid esters which contain C₆-C₁₀ fatty acids, in which the R group has between 5 and 9 carbon atoms.

Alternatively, the isosorbide ester may be prepared by a reaction of isosorbide with individual fatty acids in a highly purified form, for example a C₆ fatty acid or a C₁₀ fatty acid. The desired fatty acid ester mixture may subsequently be obtained by mixing appropriate amounts of the individual fatty acid esters. The ester may also be obtained by a reaction of isosorbide with a mixture of fatty acids, which originate from a natural fat or oil, or from a natural fat or oil which has been subjected to one or more refining steps.

In case fatty acids of natural origin are used, the fatty acid mixture used to prepare the isosorbide ester will usually be a mixture of saturated and unsaturated fatty acids, whereby preferably at least 90.0 wt.% of the fatty acids in the fatty acid mixture are saturated fatty acids, preferably at least 95.0 wt.%, more preferably at least 99.0 wt.%.

The iodine number or iodine value, expressed in grams of I₂ per gram of sample, of the isosorbide fatty acid ester of this invention is preferably smaller than 10, more preferably smaller than 5 or smaller than 1, most preferably smaller than 0.5. The iodine number is representative for the amount of unsaturated bonds present in the fatty acid ester. The limited number of unsaturated bonds in the isosorbide fatty acid ester brings the advantage that the risk for oxidation may be reduced, preferably to a minimum. It brings the advantage of a higher oxidative stability, but also a higher thermal or heat stability, of the ester but also of the pigment concentrate containing the ester, as well as any offset printing ink or varnish containing the pigment concentrate.

The applicants prefer to use fatty acids of natural origin, which are typically characterised by an even number of carbon atoms, i.e. C₄-C₂₈ fatty acids, in particular C₆-C₂₆ fatty acids. The applicants prefer to use fatty acids from natural origin, which are typically characterised by a straight chain backbone arrangement. Fatty acids which originate from a natural source will usually be available as a mixture of several fatty acids. Preferably at least 90 wt.% of the fatty acids in the mixture are straight chain fatty acids, preferably at least 95 wt.%, more preferably at least 99 wt.%. Pigment concentrates based on this type of dispersing agent are extremely suitable for use in cosmetic applications, for example white and coloured creams, powders, lipstick, or in applications where the printed material may come into contact with food.

The present invention permits providing isosorbide esters with an oral LD50 value with rats of at least 2000 mg/kg of body weight. This means in practise that a consumption of 2000 mg of the isosorbide ester of this invention per kg body weight is not considered toxic.

The amount of pigment present in the pigment concentrate of this invention may vary within wide ranges, and will usually vary from 20-90 wt. % with respect to the weight of the pigment concentrate, preferably 20-80 wt. % or 20-75 wt. %, more preferably 20-60 wt. %, most preferably 20-50 wt. %.

The amount of the above described isosorbide ester present in the pigment concentrate of this invention may vary within wide ranges and will usually vary from 10 - 50 wt.% based on the weight of the pigment concentrate, preferably from 20 - 50 wt.%, more preferably 20-40 wt. %, most preferably 20-30 wt. %.

The esterification reaction of isosorbide with at least one carboxylic acid can be carried out in a known manner, e.g. via reaction of the isosorbide possibly containing sorbitan or sorbitol with one single carboxylic acid, a suitable mixture of carboxylic acids, a suitable mixture of fatty acids or pure fatty acids. The acids can be used in stoichiometric excess with respect to the isosorbide. An excess of acid over isosorbide of from 5 to 50 mole %, more preferably from 10 to 30 mole %, is often used. The esterification reaction can be auto-catalyzed or may take place in the presence of a catalyst, for example an acidic ion exchange material. The esterification step preferably takes place by a process in which the water produced in the reaction is removed from the reaction via azeotropic distillation with the carboxylic acid.

The esterification reaction is preferably carried out in a reactor in which the reaction mixture is intensively mixed using a stirrer or of a circulating pump. The starting materials and the catalyst can be charged to the reactor simultaneously or in succession. The raw material could be used in liquid form or solid form. The isosorbide polyol is usually in the form of crystals (without water), with an isosorbide content of 97-100%. The isosorbide polyol may also be in liquid form, for example a mixture composed of 80 wt. % isosorbide and 20 wt. % water, but other mixtures may be used as well.

The isosorbide ester may be the only dispersing agent contained in the pigment concentrate. If so desired, however, one or more further dispersing agents or one ore more solvents may be present in order to fine tune the properties of the pigment concentrate, e.g. the rheological properties, the compatibility of pigment concentrate with the other components of the composition in which it is to be used, for example an offset printing ink composition or varnish composition containing the pigment concentrate, with the substrate to be printed, the rubbing resistance etc. The dispersing agent other than the isosorbide ester may vary within wide ranges and may typically represent an amount of less then 50 wt.% of the total amount of dispersing agent present, preferably less then 25 wt.%.

The present invention further provides for new methods for producing a pigment concentrate and for producing an offset printing ink, which methods involve the incorporation of the above-described isosorbide ester in a pigment concentrate.

The present invention also relates to a composition containing the above described pigment concentrate. The present invention particularly relates to an offset printing ink and an offset varnish composition comprising the above described pigment concentrate, in particular an offset printing ink. The present invention also relates to a cosmetic composition comprising the above described pigment concentrate, in particular a cream, ointment or gel, a powder, lipstick, nail polish.

The present invention also relates to the use of the above described pigment concentrate for producing an offset printing composition or an offset varnish composition suitable for use with food packaging, preferably with primary food packaging.

The present invention further relates to a method for improving the colour strength and/or colour density provided by an ink composition, in particular an offset printing ink composition, wherein the ink composition contains an amount of the above described pigment concentrate.

The present invention further relates to a method for improving the gloss produced by an ink composition upon printing, in particular an offset printing ink composition, wherein the ink composition contains an amount of the above described pigment concentrate.

The present invention also relates to a cosmetic composition, in particular a cream, ointment, gel, lotion or wax comprising the pigment concentrate of the present invention. The present invention further relates to a method for improving the colour strength and/or colour density and/or gloss of a cosmetic composition, in particular a cream, ointment, gel, lotion or wax comprising the pigment concentrate of the present invention.

### PIGMENT.

The concentration of the solid pigment in the pigment concentrate may vary within a relatively wide range, and will usually be at least 20 wt. % with respect to the total weight of the pigment concentrate, preferably at least 25 wt. % or 30 wt. %, more preferably at least 35.0 wt. % or 40 wt. % although concentrations above 50.0 wt. %, preferably above 55.0 wt. % or 60.0 wt. % may be used as well. The concentration of the solid pigment in the pigment concentrate will usually not be higher than 90 wt. %, and will preferably be below 85 wt. %, more preferably below 80 wt. %, most preferably below 75 wt. %. Depending on the nature of the envisaged application and the nature of the pigment, the pigment concentration may be lower than 65 wt. %, even lower than 60 wt. % or below 50 wt. %.

Within the scope of the present invention, a pigment includes any colouring material that forms a heterogeneous mixture with the carrier of an ink or varnish composition at normal process conditions. The skilled person will be capable of selecting the appropriate pigment depending on the envisaged ink colour. Examples of pigments suitable for use with the present invention include carbon black, titanium dioxide, phthalocyanines such as copper pthalocyanine blue, azo pigments, quinacridones, anthraquinones, dioxazines, indigos, thio-indigos, perynones, perylenes, indolenones, azomethines, triphenyl methanes, and the like. Dry pigments that are not available in press cake form may be reconstituted with water.

Many pigments are commercially available as wet press cakes, which may be re-dispersed in solution to form an aqueous pigment slurry if desired. Examples of commercially available pigments include Copper Phthalocyanine Blue G/S press cake (BL2101-PC) (37.1% by weight pigment content); Lithol Rubine press cake (LR5133-PC) (21.6% by weight pigment content); and Diarylid Yellow AAA T/P press cake (YA1933-PC) (18.0% by weight pigment content), all available from Magruder Colour Co., Inc., Elizabeth, N.Y. Some manufacturers also will provide aqueous slurries of pigment particles.

Other commercially available pigments and press cakes include those of the following pigments: Phtalocyanine, Pigment red, Diaryl Yellow, Metallized Azo Reds: Red 49:1 (Barium salt), Red 49:2 (Calcium salt), Red 63:1 (Calcium salt), Toluidine reds, Naphthol reds, Pyrazolones, Rhodamines, Quiacridones: Red B, Red Y, Magenta B, Magenta and violet, Phthalocyanine blues, Phthalocyanine greens, Carbazole violets, Monoarylid Yellow, Diarylid Yellow, Chrome yellow, Red Lake c, Lithol reds: calcium and barium salts Bon Maroon, Perylene pigments, Red 2B: Calcium, Barium and Magnesium salts. Alkali Blue, Chrome Orange, Molybdate orange, Orange 36, Diarylide orange, Dianisidine orange, tolyl orange and Dinitraniline orange.

Such pigments may have a wide range of particle sizes, from about 0.04 to about 100 µm, preferably from about 0.04 to about 50 µm, and more preferably from about 0.04 µm to about 20 µm or 10 µm. Most preferably, the pigment particle size is from about 0.04 µm to about 10 µm when used in an ink. If desired, the pigment particles can be milled, although this is not necessary to the practices of the invention.

Methods for producing printing ink concentrates are for example disclosed in EP-A-807.670.

### VARNISH.

The pigment concentrate of the present invention may comprise a varnish composition that is compatible with the pigment particles.

The varnish composition may further include an amount of the isosorbide ester dispersing agent as described above, a dispersing polymer for the solid pigment particles, a hydrophobic carrier, a supporting resin, a wetting polymer, and optionally any other usual ingredients such as one ore more rheological-property modifying agents, including softeners, a plasticizer, an antioxidant etc.

The presence of the dispersing polymer further assists in counteracts re-agglomeration of the solid pigment particles and helps to stabilize the distribution of the pigment particles. The concentration of the dispersing polymer in the varnish will often vary from about 1.0 wt. % to about 15.0 wt. % by weight; preferably from 3 wt. % to about 8 wt. % by weight of the varnish.

The supporting resin comprises a wetting agent that will wet the surface of the pigment particles and provide support for the pigment particles when they are released onto a printing substrate. The supporting resin preferably is included in an amount from about 5.0 wt. % to about 35 wt. % by weight of the varnish, preferably from about 10.0 to about 25.0 wt %. Examples of supporting resins suitable for use with the present invention include rosin esters which usually originate from pine trees and have been chemically modified for example phenolic modified rosin ester (Hercules, Wilmington, Del) hydrocarbon resins modified rosin esters, terpenic resins, and wood rosin resins, a terpene hydrocarbon resin, a modified hydrocarbon resin.

In order to minimize the risk to deteriorating colour upon storage of the pigment concentrate, a the wetting polymer may be incorporated into the varnish, that assists in wetting the surface of the pigment particles. Examples of wetting polymers suitable for use with this invention include acrylic resins, polyolefins, acrylic polymers, polyesters and urethane polymers, and the like, as well as copolymers, and graft polymers of the aforementioned, as well as modified polymers of the afore-mentioned polymers to have surface active groups such as hydroxyl, carboxyl, carbonyl, amino, ammonium, or nitro groups, or the like.

An antioxidant may be included in an amount effective to provide an ant oxidizing effect on the hydrophobic carrier. Examples of suitable antioxidants include tocopherol, hydroquinones, and butylated hydroxytoluene. The varnish further may include a plasticizer, such as a low molecular weight polyester. Additional light oil diluents may be used, if desired.

### PREPARATION OF THE PIGMENT CONCENTRATE

To produce the pigment concentrate, a slurry of pigment particles is incorporated into the isosorbide ester described above as dispersing agent. Suitably a ratio of about 1:10 to about 10:1 by weight of the pigment particles in relation to the varnish may be used; preferably, a ratio of about 3:7 to about 4:6 by weight of the pigment particles. It is advisable to ensure good wetting of the pigment particles by the ester, as this favours the stability of the pigment dispersion.

A pigment concentrate may also be produced by blending dry pigment particles with a varnish which contains the isosorbide ester dispersing agent described above. The blend is heated to melt the dispersing agent, which upon cooling solidifies on the surface of the pigment particles to wet the particles and prevent them from agglomerating. With this procedure it is advised to vigorously stir the mixture after adding the varnish to the pigment particles, at an elevated temperature, to allow the varnish to bind more readily to the pigment particles.

### PREPARATION OF AN OFFSET INK.

An offset ink may be prepared from the above described pigment concentrate by blending an amount of the pigment concentrate with a further ink vehicle. Any vehicle typically used for lithographic (direct and offset), letterpress, gravure, flexographic, silk-screen, or mimeograph printing processes, or vehicles used to form curable inks, such as infrared, ultraviolet and electron beam (E.B.) curable inks may be used.

Generally the pigments will be present in the offset printing ink in an amount from about 0.5 to about 30 percent (dispersion agent excluded), based on the total weight of the ink and based on a dry pigment. Other suitable pigment concentrations, based on dry pigment weight, may range from 0.5 - 15.0 wt. % of the ink composition, preferably from 0.5 - 10.0 wt. % or 0.5-5 wt. %. The inventors have namely observed that the pigment concentrate of this invention is capable of increasing colour strength.

Any ink vehicle considered suitable by the skilled person, may be employed. An example of a vehicle suitable for use in offset printing comprises an oil base, a modified rosin ester, an anti-blocking agent, a thixotropic agent and dryer. The ink may be prepared by mixing the pigment concentrate with the further components of the ink described above. An ink thus prepared will exhibit excellent transfer and will produce high quality prints with high colour intensity and high gloss.

The present invention is further illustrated in the examples below.

In the examples below the particle size distribution of the pigment particles was measured using a Malvern Mastersizer 2000.

Gloss was measured with a Novo-Gloss Trigloss meter at three different positions of a sample, according to ASTM D523. Gloss is an aspect of the visual perception of a printed surface that is as important as colour. Gloss was measured using the reference measurement angle of 60°, as well as the complementary angles of 20 and 85°. The 20° angle gives improved resolution for high gloss surfaces, and is more sensitive to Haze effects, whereas the 85° angle gives improved resolution for matt finishes.

Where indicated below, mixing was carried out using a Muller type mixer, which incorporates kneading, sharing, smearing and blending of material to achieve good consistency. An amount of raw material is spread over a limited area of the glass plate. The mixture is ground in successive stages, for example 50 revolutions each, at a pressure, of 445N. Between each stage, the mixture is be redistributed on the glass plate. After respectively the first, second and third pass a small quantity of the pigment concentrate was taken and its dispersion properties were evaluated using a Hegman gauge.

The Hegman gauge is a flat steel block in the surface of which are two flat-bottomed grooves varying uniformly in depth from a maximum at one end of the block to zero near the other end. Groove depth is graduated on the block according to one or more scales used for measuring particle size. The Fineness of Grind Gage is used to indicate the fineness of grind or the presence of coarse particles or agglomerates in a dispersion. It does not determine particle size or particle size distribution. The physical properties of a dispersions, depend not only on the actual size of the individual particles, but also on the degree to which they are dispersed.

The colour of a homogeneous film of the pigment concentrate was measured using the drawdown test. First, a film was prepared by spreading ink with over a substrate using a film applicator over a drawdown test card. The drawdown card consists of black and white areas with sufficient room to use colour measurement instruments to measure properties such as the colour, opacity, ..., of ink sample. Thereby use was made of a film applicator in the form of a metal bar manufactured to high tolerance to produce films with consistent, repeatable results. The bar gives a theoretical wet film thickness, the thickness of the coating that should remain on the drawdown card after application. Upon drying, the film thickness usually decreases somewhat. The film however remained uniform and homogeneous.

The colour and lightness of a thus formed homogeneous film was further measured using a spectrophotometer, in particular the CIELab system. The system consists of two axes a* and b* which are at right angles and represent the hue dimension or colour. The third axis, perpendicular to a* and b* is the lightness L*. Within this system, any colour can be specified with the coordinates L*, a*, b*. C* (= Chroma) represents the intensity or saturation of the colour. This colorimetric system can be visualized by a three-dimensional colour space, where every colour can be represented by a set of 3 co-ordinates: L*, a* and b*, where L* indicates the lightness and a* and b* the chromaticity coordinates. Positive values of a* show the red direction, and negative values the green direction, whereas positive values of b* show the yellow direction and negative values the blue direction. The center is achromatic (i.e. neutral).

### Comparative experiment A.

A pigment concentrate was produced by mixing 50 parts by weight of a standard varnish composition with 30 parts by weight of respectively a blue pigment 15:3, and a black pigment 7:30, 8 parts of soya oil, 12 parts of mineral turpentine oil, until the pigment is fully wetted. The mixture was mixed with a spatula until complete wetting of the pigment.

Thereafter, the mixture was passed on to a triple roll Muller mill by mixing for 1 min on 2 rolls. The mill had a roll contact pressure of 20 kg/cm² during mixing. Thereafter, the thus obtained mixture was passed over the triple roll mill, in a sequence of three passes, while maintaining a roll contact pressure of 25 kg/cm². After respectively the first, second and third pass a small quantity of the pigment concentrate was taken and its dispersion properties were evaluated using a Hegman gauge as described above. The results of this measurement are shown in Table 1.

### Comparative experiment B - Preparation of an ink containing 17 wt. % of pigment concentrate.

56.6 parts of the 30 % ink concentrate of Comparative experiment A were mixed with 28.4 parts of grinding medium and 10.0 parts of letdown varnish, 2 parts of soy oil and 3 parts of mineral turpentine oil. The mixture was mixed with a knife for 3-5 minutes and further in a Muller with small pressure as described above.

The particle size distribution of the thus obtained ink composition is summarized in table 3.

The gloss was measured as described above. The results are summarized in table4.

The colour strength was measured as described above. The results are summarized in table 5.

### Comparative experiment C - Preparation of an ink containing 4 wt. % of pigment concentrate.

The ink produced in Comparative experiment B was further diluted with the standard letdown varnish, until a pigment concentration of 4 wt. % was obtained.

The colour strength was measured as described above. The results are summarized in table 5.

### Example 1 - Preparation of isosorbide ester.

A reaction vessel was charged with pure isosorbide having a purity of 99 %. A fatty acid mixture consisting 55 wt. % of n-octanoic acid and 45 wt. % of n-decanoic acid was added in an excess to isosorbide, in a weight ratio of 1.90 equivalents of fatty acid per equivalent of isosorbide. 1.90 wt. % with respect to the weight of the isosorbide of an acid esterification catalyst was added. The esterification reaction was carried out at elevated temperature and was stopped before an acid value of 5 mg KOH/g was obtained.

The crude reaction product contained a blend of fatty acid isosorbide mono-ester, fatty acid isosorbide di-ester and an excess of fatty acid that had not reacted. The excess of fatty acid was removed from the reaction mixture by distillation under vacuum (<40 mbar), the temperature was gradually increased from 150°C to 210°C. The reaction mixture was neutralized by addition of a base. The reaction mixture was stripped with steam at 210°C under vacuum.

The surface tension, Kauri butanol value and physicochemical characteristics of the fatty acid isosorbide ester thus produced are given in Table 2 .

### Example 2. Preparation of a pigment concentrate containing 30 wt. % of pigment.

The procedure described in comparative example A for preparing a pigment concentrate was repeated, but in stead of soya oil and turpentine oil, use was made of the isosorbide ester produced according to example 1.

Thereto, a pigment concentrate was produced by mixing 50 parts by weight of a standard varnish composition with 30 parts by weight of respectively a blue pigment 15:3, and a black pigment 7:30, 20 parts of the isosorbide ester, until the pigment is fully wetted. The mixture was mixed with a spatula until complete wetting of the pigment. Thereafter, the mixture was passed on to a triple roll mill by mixing for 1 min on 2 rolls. The mill had a roll contact pressure of 20 kg/cm² during mixing. Thereafter, the thus obtained mixture was passed over the triple roll mill, in a sequence of three passes, while maintaining a roll contact pressure of 25 kg/cm2.

After respectively the first, second and third pass a small quantity of the pigment concentrate was taken and its dispersion properties were evaluated. The results are shown in table 1.

From table 1 it can be observed that the use of the isosorbide ester as dispersing agent permits to obtain pigment particles in a dispersion with a finer degree of grinding, when compared to preparing the same dispersion in the prior art dispersing agent. This has been observed both in respect of the blue and the black pigment.

### Example 3 - Preparation of an ink containing 17 wt. % of pigment concentrate.

56.6 parts of the 30 % ink concentrate of example 2 were mixed with 28.4 parts of grinding medium and 10.0 parts of letdown varnish, 2 parts of soy oil and 3 parts of mineral turpentine oil. The mixture was mixed with a knife for 3-5 minutes and further in a Muller with small pressure as described below.

From table 1 it can be observed that the use of the isosorbide ester as dispersing agent permits to obtain pigment particles in a dispersion with a degree of grinding which is at least as good as that obtained with the prior art dispersing agent. This has been observed both in respect of the blue and the black pigment. The present invention thus provides a dispersion agent which is fully made of renewable materials and which is capable of providing and maintaining a degree of grinding of the pigment that is at least comparable to the existing dispersion agent.

The particle size distribution of the thus obtained ink composition is summarized in table 3. From table 3 it can be observed that with the dispersion agent of the present invention a dispersion of the blue and black pigment may be obtained, in which the pigment particles have a smaller size as compared to the prior art dispersion agent. It may thus be concluded that the dispersion agent of the present invention shows improved wetting and deflocculating properties. As a result hereof, colour strength of the pigment may be improved, in particular with increasing dilution of the pigment in the ink composition. This is supported by the data of table 5, which show the colour strength of an ink composition containing respectively 17 wt. % and 4 wt. % of pigment.

The gloss was measured as described above. The results are summarized in table 4. From table 4 it may be concluded that an ink composition which contains the pigment concentrate of this invention with a dispersion agent made of renewable materials provides gloss properties which are comparable to the prior art.

The Colour Coordinates were measured according to the CIELab system described above. The results are summarized in table 5. From table 5 it appears that Chroma or colour saturation obtained when using the dispersion agent of this invention is comparable to the Chroma obtained with the prior art system.

### Example 4 - Preparation of an ink containing 4 wt. % of pigment concentrate.

The ink produced in example 3 was further diluted with the isosorbide ester of example 1, until a pigment concentration of 4 wt. % was obtained.
The colour strength was measured as described above. The results are summarized in table 5.

**Table 1. Fineness of grinding (µm) of the pigment particles using Hegman Gauge.**

| | Black Pigment | | | | Blue Pigment | | | |
|---|---|---|---|---|---|---|---|---|
| Particle size (µm) | Comp. exp. A | Ex. 2 | Comp. Exp. B | Ex. 3 | Comp. exp. A | Ex. 2 | Comp. Exp. B | Ex. 3 |
| After 1^{st} grinding | 25.0 | 25.0 | | | 25.0 | 25.0 | | |
| After 2^{nd} grinding | 22.5 | 17.5 | | | 17.5 | 10.0 | | |
| After 3^{rd} grinding | 7.5 | 7.5 | 7.5 | 7.5 | 12.5 | 8.0 | 7.5 | 7.5 |

**Table 2 : Physicochemical properties of an ester of isosorbide and a mixture of 55 wt. % of n-octanoic acid and 45 wt. % of n-decanoic acid.**

| | |
|---|---|
| Kauri Butanol ASTM D1133 | 96 |
| Density (g/cm3) at 25°C | 1.019 |
| Molecular wt. (calculated) | 420 |
| Acid value (mg KOH/g) AOCS Cd 3d-63 | 0.78 |
| wt.% mono-ester (calculated) | 3.98 |
| OH-value (mg KOH/g) AOCS Cd 13-60 | 8.2 |
| Colour (APHA) AOCS Tdlb-64 | 166 |
| Dynamic viscosity at 25°C (mPa.s) | 64.3 |
| Pour point (°C) ASTM D97 | -6°C |
| Flash point (°C) AOCS Cc9a-48 | |
| Odour before stripping | Faint |

**Table 3. Particle size distribution of an ink containing 17 wt. % of pigment.**

| | Particle size distribution Blue Pigment 15:3 | | | | Particle size distribution Black Pigment 7 | | | |
|---|---|---|---|---|---|---|---|---|
| Pigment concentrate | D10 | D50 | D90 | Span | D10 | D50 | D90 | Span |
| Comp. exp. A | 0.74 | 2.15 | 7.17 | 2.99 | 0.68 | 1.76 | 8.31 | 4.33 |
| Ex. 2 | 0.69 | 1.77 | 6.65 | 3.36 | 0.65 | 1.47 | 7.83 | 4.88 |

**Table 4. Gloss of an ink containing 17 wt. % of pigment.**

| Gloss at | Black Pigment 7 | Blue Pigment 15:3 | Black Pigment 7 | Blue Pigment 15:3 |
|---|---|---|---|---|
| | Ex. 3 | Ex. 3 | Comp. exp. B | Comp. exp. B |
| 20° | 0.2 | 0.2 | 0.2 | 0.2 |
| 60° | 4.1 | 3.1 | 3.4 | 3.3 |
| 85° | 3.7 | 3.7 | 3.7 | 3.6 |

**Table 5. Colour strength and Chroma.**

| | Colour strength | | Chroma C | |
|---|---|---|---|---|
| | Black pigment 7 | Blue pigment 15:3 | Black pigment 7 | Blue pigment 15:3 |
| Ex. 2 | | | | 29.175 |
| Comp. Exp. A | | | | 31.155 |
| Ex. 3 | 101.48 % | 94.11 % | 2.741 | 34.249 |
| Comp. exp. B | 100.00 % | 100.00 % | 3.044 | 36.194 |
| Ex. 4 | 201.42 % | 138.82 % | 2.218 | 34.971 |
| Comp. exp. C | 100.00 % | 100.00 % | 1.242 | 31.664 |

## Claims

1. Use of a monomeric ester of a polyol with at least one carboxylic according to formula I 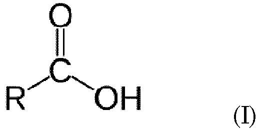 wherein R is a hydrocarbon moiety containing from 3 up to 27 carbon atoms, wherein the polyol comprises isosorbide and the average degree of esterification of the polyol moieties in the isosorbide ester ranges from 1.50 to 1.99, more preferably from 1.60 to 1.98, in particular from 1.70 to 1.97, more in particular from 1.75 to 1.95 and wherein the monomeric ester is a mixture comprising monoesters and polyesters of isosorbide, which mixture comprises isosorbide monoester at a level of at most 50 wt.% based on the total weight of the ester mixture, preferably at most 30 wt.% monoester, more preferably at most 20 wt.% and even more preferably at most 10 wt.% monoester, as a dispersing agent for the preparation of a pigment concentrate of particles of one or more pigments.

2. Use according to claim 1, wherein the polyol consists of isosorbide.

3. Use according to any one of the previous claims wherein the monomeric ester has a Kauri-butanol value of at least 25, preferably at least 50, more preferably at least 60, even more preferably at least 70, yet more preferably at least 80.

4. Use according to claim 3, wherein the monomeric ester has a Kauri-butanol value of at most 170, preferably at most 160, more preferably at most 150 and even more preferably at most 140.

5. Use according to any one of the previous claims wherein the ester has a hydroxyl value of at most 50 mg KOH/g, preferably at most 40 mg KOH/g, more preferably at most 30 mg KOH/g and most preferably at most 20.0 mg KOH/g.

6. Use according to any one of the previous claims wherein the ester has a hydroxyl value of at least 5.0 mg KOH/g, preferably at least 8.0 mg KOH/g, more preferably at least 10.0 mg KOH/g and most preferably at least 15.0 mg KOH/g.

7. Use according to any one of the previous claims wherein the ester is a mixture comprising monoesters and polyesters of isosorbide, which mixture comprises isosorbide monoester at a level of at least 1.0 wt.% based on the total weight of the ester mixture, preferably at least 3.0 wt.% monoester, more preferably at least 4.0 wt.% and even more preferably at least 5.0 wt.% monoester.

8. Use according to any one of the previous claims wherein the monomeric ester has a dynamic viscosity at 25°C in the range of 20-600 mPa.s, preferably 40-400 mPa.s, more preferably 40-300 mPa.s, more preferably 40-200 mPa.s and most preferably 40- 100 mPa.s.

9. Use according to any one of the previous claims wherein the at least one carboxylic acid is a fatty acid mixture which comprises at least 75 wt.% of C₄-C₁₂ fatty acids, more preferably at least 85 wt.%, even more preferably at least 90.0 wt.%, yet more preferably at least 95.0 wt.% based on the fatty acid weight.

10. Use according to any one of the previous claims wherein the at least one fatty acid is a mixture which comprises at most 15 wt.% of C₁₂-C₂₂ fatty acids with respect to the fatty acid weight, preferably at most 10.0 wt.%, more preferably at most 5.0 wt.%, most preferably at most 3.0 wt.%.

11. Use according to any one of the previous claims wherein the at least one fatty acid is a mixture which comprises at most 5.0 wt.% with respect to the fatty acid weight of C₁₂-C₁₈ fatty acids, preferably at most 3.0 wt.%.

12. Use according to any one of the previous claims, wherein at least 90 wt. % of the carboxylic acids of the at least one carboxylic acid are saturated carboxylic acids, preferably at least 95 wt.%, more preferably at least 99 wt.%.

13. Use according to any one of the previous claims wherein the ester has an iodine number or iodine value, expressed in grams of I₂ per gram of ester, which is smaller than 10, preferably smaller than 5, more preferably smaller than 1, most preferably smaller than 0.5.

14. Use according to any one of the previous claims wherein at least 90 wt.% of the at least one carboxylic acid are straight chain carboxylic acids, preferably at least 95 wt.%, more preferably at least 99 wt.%.

15. A pigment concentrate comprising particles of one or more pigments in an amount of at least 30 wt% dispersed in at least one dispersing agent **characterised in that** the at least one dispersing agent comprises a monomeric ester of a polyol with at least one carboxylic according to formula I 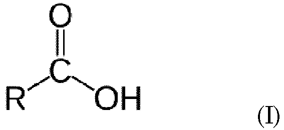 wherein R is a hydrocarbon rest containing from 3 up to 27 carbon atoms, wherein the polyol comprises isosorbide and the average degree of esterification of the polyol moieties in the isosorbide ester ranges from 1.50 to 1.99, more preferably from 1.60 to 1.98, in particular from 1.70 to 1.97, more in particular from 1.75 to 1.95 and wherein the monomeric ester is a mixture comprising monoesters and polyesters of isosorbide, which mixture comprises isosorbide monoester at a level of at most 50 wt.% based on the total weight of the ester mixture, preferably at most 30 wt.% monoester, more preferably at most 20 wt.% and even more preferably at most 10 wt.% monoester.

16. Use of a pigment concentrate obtained according to any one of claims 1-14 or the pigment concentrate of claim 15 for the preparation of an offset printing ink composition.

17. Use according to claim 16 for the preparation of an offset printing ink composition or an offset varnish composition suitable for use with food packaging, preferably with primary food packaging.

18. Use of a pigment concentrate obtained according to any one of claims 1-14 or the pigment concentrate of claim 15, for improving the colour strength and/or colour density and/ or gloss of an offset printing ink composition or an offset varnish composition.

19. Use of a pigment concentrate obtained according to any one of claims 1-14 or the pigment concentrate of claim 15, for the preparation of a cosmetic composition, in particular a cream, ointment, gel, lotion or wax.

## Patentansprüche

1. Verwendung eines monomeren Esters eines Polyols mit wenigstens einer Carbonsäure gemäß Formel I 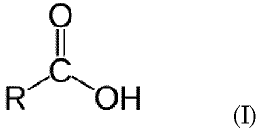 wobei R ein Kohlenwasserstoffrest ist, enthaltend von 3 bis zu 27 Kohlenstoffatome, wobei das Polyol Isosorbid umfasst und der durchschnittliche Veresterungsgrad der Polyolreste in dem Isosorbidester sich von 1,50 bis 1,99, bevorzugter von 1,60 bis 1,98, insbesondere von 1,70 bis 1,97, insbesonderer von 1,75 bis 1,95 bewegt und wobei der monomere Ester eine Mischung ist, umfassend Monoester und Polyester von Isosorbid, wobei die Mischung Isosorbidmonoester in einer Höhe von höchstens 50 Gew.-%, basierend auf dem Gesamtgewicht der Estermischung, vorzugsweise höchstens 30 Gew.-% Monoester, bevorzugter höchstens 20 Gew.-% und noch bevorzugter höchstens 10 Gew.-% Monoester, als Dispersionsmittel für die Herstellung eines Pigmentkonzentrats aus Partikeln aus einem oder mehreren Pigmenten umfasst.

2. Verwendung gemäß Anspruch 1, wobei das Polyol aus Isosorbid besteht.

3. Verwendung gemäß einem der vorhergehenden Ansprüche, wobei der monomere Ester eine Kauri-Butanol-Zahl von wenigstens 25, vorzugsweise wenigstens 50, bevorzugter wenigstens 60, noch bevorzugter wenigstens 70, sogar noch bevorzugter wenigstens 80 hat.

4. Verwendung gemäß Anspruch 3, wobei der monomere Ester eine Kauri-Butanol-Zahl von höchstens 170, vorzugsweise höchstens 160, bevorzugter höchstens 150 und noch bevorzugter höchstens 140 hat.

5. Verwendung gemäß einem der vorhergehenden Ansprüche, wobei der Ester eine Hydroxylzahl von höchstens 50 mg KOH/g, vorzugsweise höchstens 40 mg KOH/g, bevorzugter höchstens 30 mg KOH/g und am bevorzugtesten höchstens 20,0 mg KOH/g hat.

6. Verwendung gemäß einem der vorhergehenden Ansprüche, wobei der Ester eine Hydroxylzahl von wenigstens 5,0 mg KOH/g, vorzugsweise wenigstens 8,0 mg KOH/g, bevorzugter wenigstens 10,0 mg KOH/g und am bevorzugtesten wenigstens 15,0 mg KOH/g hat.

7. Verwendung gemäß einem der vorhergehenden Ansprüche, wobei der Ester eine Mischung ist, umfassend Monoester und Polyester von Isosorbid, wobei die Mischung Isosorbidmonoester in einer Höhe von wenigstens 1,0 Gew.-%, basierend auf dem Gesamtgewicht der Estermischung, vorzugsweise wenigstens 3,0 Gew.-% Monoester, bevorzugter wenigstens 4,0 Gew.-% und noch bevorzugter wenigstens 5,0 Gew.-% Monoester umfasst.

8. Verwendung gemäß einem der vorhergehenden Ansprüche, wobei der monomere Ester eine dynamische Viskosität bei 25 °C in dem Bereich von 20 - 600 mPa.s, vorzugsweise 40 - 400 mPa.s, bevorzugter 40 - 300 mPa.s, bevorzugter 40 - 200 mPa.s und am bevorzugtesten 40 - 100 mPa-s hat.

9. Verwendung gemäß einem der vorhergehenden Ansprüche, wobei die wenigstens eine Carbonsäure eine Fettsäuremischung ist, die wenigstens 75 Gew.-% C₄-C₁₂ Fettsäuren, bevorzugter wenigstens 85 Gew.-%, noch bevorzugter wenigstens 90,0 Gew.-%, sogar noch bevorzugter wenigstens 95,0 Gew.-%, basierend auf dem Fettsäuregewicht, umfasst.

10. Verwendung gemäß einem der vorhergehenden Ansprüche, wobei die wenigstens eine Fettsäure eine Mischung ist, die höchstens 15 Gew.-% von C₁₂-C₂₂ Fettsäuren in Bezug auf das Fettsäuregewicht, vorzugsweise höchstens 10,0 Gew.-%, bevorzugter höchstens 5,0 Gew.-%, am bevorzugtesten höchstens 3,0 Gew.-% umfasst.

11. Verwendung gemäß einem der vorhergehenden Ansprüche, wobei die wenigstens eine Fettsäure eine Mischung ist, die höchstens 5,0 Gew.-% in Bezug auf das Fettsäuregewicht von C₁₂-C₁₈ Fettsäuren, vorzugsweise höchstens 3,0 Gew.-%, umfasst.

12. Verwendung gemäß einem der vorhergehenden Ansprüche, wobei wenigstens 90 Gew.-% der Carbonsäuren der wenigstens einen Carbonsäure gesättigte Carbonsäuren sind, vorzugsweise wenigstens 95 Gew.-%, bevorzugter wenigstens 99 Gew.-%.

13. Verwendung gemäß einem der vorhergehenden Ansprüche, wobei der Ester eine Iodnummer oder eine Iodzahl, ausgedrückt in Gramm von I₂ pro Gramm Ester, hat, die kleiner als 10, vorzugsweise kleiner als 5, bevorzugter kleiner als 1, am bevorzugtesten kleiner ist als 0,5 ist.

14. Verwendung gemäß einem der vorhergehenden Ansprüche, wobei wenigstens 90 Gew.-% der wenigstens einen Carbonsäure geradkettige Carbonsäuren sind, vorzugsweise wenigstens 95 Gew.-%, bevorzugter wenigstens 99 Gew.-%.

15. Pigmentkonzentrat, umfassend Partikel eines oder mehrerer Pigmente in einer Menge von wenigstens 30 Gew.-%, dispergiert in wenigstens einem Dispersionsmittel, **dadurch gekennzeichnet, dass** das wenigstens eine Dispersionsmittel einen monomeren Ester eines Polyols mit wenigstens einer Carbonsäure gemäß Formel I 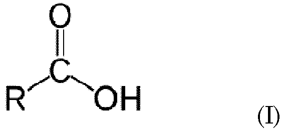 umfasst, wobei R ein Kohlenwasserstoffrest ist, enthaltend von 3 bis zu 27 Kohlenstoffatome, wobei das Polyol Isosorbid umfasst und der durchschnittliche Veresterungsgrad der Polyolreste in dem Isosorbidester sich von 1,50 bis 1,99, bevorzugter von 1,60 bis 1,98, insbesondere von 1,70 bis 1,97, insbesonderer von 1,75 bis 1,95 bewegt, und wobei der monomere Ester eine Mischung ist, umfassend Monoester und Polyester von Isosorbid, wobei die Mischung Isosorbidmonoester in einer Höhe von höchstens 50 Gew.-%, basierend auf dem Gesamtgewicht der Estermischung, vorzugsweise höchstens 30 Gew.-% Monoester, bevorzugter höchstens 20 Gew.-% und noch bevorzugter höchstens 10 Gew.-% Monoester umfasst.

16. Verwendung eines Pigmentkonzentrats, erhalten gemäß einem der Ansprüche 1 - 14 oder des Pigmentkonzentrats nach Anspruch 15 zur Herstellung einer Offsetdruckfarbenzusammensetzung.

17. Verwendung gemäß Anspruch 16 zur Herstellung einer Offsetdruckfarbenzusammensetzung oder einer Offsetlackzusammensetzung, geeignet zur Verwendung mit Lebensmittelverpackung, vorzugsweise mit primärer Lebensmittelverpackung.

18. Verwendung eines Pigmentkonzentrats, erhalten gemäß einem der Ansprüche 1 - 14 oder des Pigmentkonzentrat nach Anspruch 15 zum Verbessern der Farbstärke und/oder Farbdichte und/oder des Glanzes einer Offsetdruckfarbenzusammensetzung oder einer Offsetlackzusammensetzung.

19. Verwendung eines Pigmentkonzentrats, erhalten gemäß einem der Ansprüche 1 - 14 oder des Pigmentkonzentrat nach Anspruch 15, zur Herstellung einer kosmetischen Zusammensetzung, insbesondere einer Creme, Salbe, eines Gels, einer Lotion oder eines Wachses.

## Revendications

1. Utilisation d'un ester monomère d'un polyol avec au moins un groupe carboxylique selon la formule I 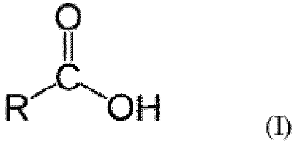 dans laquelle R est une moitié hydrocarbonée contenant de 3 jusqu'à 27 atomes de carbone, dans laquelle le polyol comprend de l'isosorbide et le degré moyen d'estérification des moitiés polyol dans l'ester d'isosorbide est de 1,50 à 1,99, de préférence de 1,60 à 1,98, en particulier de 1,70 à 1,97, encore mieux de 1,75 à 1,95 et dans laquelle l'ester monomère est un mélange comprenant des monoesters et polyesters d'isosorbide, lequel mélange comprend un monoester d'isosorbide à un niveau d'au plus 50 % en masse rapporté à la masse totale du mélange d'esters, de préférence d'au plus 30 % en masse de monoester, encore mieux d'au plus 20 % en masse et bien mieux encore d'au plus 10 % en masse de monoester, comme un agent dispersant pour la préparation d'un concentré de pigment de particules d'un ou plusieurs pigments.

2. Utilisation selon la revendication 1, dans laquelle le polyol consiste en isosorbide.

3. Utilisation selon l'une quelconque des revendications précédentes, dans laquelle l'ester monomère présente un indice Kauri butanol d'au moins 25, de préférence d'au moins 50, encore mieux d'au moins 60, bien mieux encore d'au moins 70, particulièrement de préférence d'au moins 80.

4. Utilisation selon la revendication 3, dans laquelle l'ester monomère présente un indice Kauri butanol d'au plus 170, de préférence d'au plus 160, encore mieux d'au plus 150 et bien mieux encore d'au plus 140.

5. Utilisation selon l'une quelconque des revendications précédentes, dans laquelle l'ester présente un indice hydroxyle d'au plus 50 mg de KOH/g, de préférence d'au plus 40 mg de KOH/g, bien mieux encore d'au plus 30 mg de KOH/g et particulièrement de préférence d'au plus 20,0 mg de KOH/g.

6. Utilisation selon l'une quelconque des revendications précédentes, dans laquelle l'ester présente un indice hydroxyle d'au moins 5,0 mg de KOH/g, de préférence d'au moins 8,0 mg de KOH/g, encore mieux d'au moins 10,0 mg de KOH/g et bien mieux encore d'au moins 15,0 mg de KOH/g.

7. Utilisation selon l'une quelconque des revendications précédentes, dans laquelle l'ester est un mélange comprenant des monoesters et polyesters d'isosorbide, lequel mélange comprend un monoester d'isosorbide à une teneur d'au moins 1,0 % en masse rapporté à la masse totale du mélange d'esters, de préférence d'au moins 3,0 % en masse de monoester, encore mieux d'au moins 4,0 % en masse et bien mieux encore d'au moins 5,0 % en masse de monoester.

8. Utilisation selon l'une quelconque des revendications précédentes, dans laquelle l'ester monomère présente une viscosité dynamique à 25°C dans l'intervalle de 20-600 mPa.s, de préférence 40-400 mPa.s, encore mieux 40-300 mPa.s, bien mieux encore 40-200 mPa.s et particulièrement de préférence 40-100 mPa.s.

9. Utilisation selon l'une quelconque des revendications précédentes, dans laquelle le au moins un acide carboxylique est un mélange d'acides gras qui comprend au moins 75 % en masse d'acides gras en C₄-C₁₂, encore mieux au moins 85 % en masse, bien mieux encore au moins 90,0 % en masse, particulièrement de préférence au moins 95,0 % en masse rapporté à la masse d'acides gras.

10. Utilisation selon l'une quelconque des revendications précédentes, dans laquelle le au moins un acide gras est un mélange qui comprend au plus 15 % en masse d'acides gras en C₁₂-C₂₂ par rapport à la masse d'acides gras, de préférence au plus 10,0 % en masse, encore mieux au plus 5,0 % en masse, particulièrement de préférence au plus 3,0 % en masse.

11. Utilisation selon l'une quelconque des revendications précédentes, dans laquelle le au moins un acide gras est un mélange qui comprend au plus 5,0 % en masse par rapport à la masse d'acides gras d'acides gras en C₁₂-C₁₈, de préférence au plus 3,0 % en masse.

12. Utilisation selon l'une quelconque des revendications précédentes, dans laquelle au moins 90 % en masse des acides carboxyliques du au moins un acide carboxylique sont des acides carboxyliques saturés, de préférence au moins 95 % en masse, encore mieux au moins 99 % en masse.

13. Utilisation selon l'une quelconque des revendications précédentes, dans laquelle l'ester présente un indice d'iode ou valeur diode, exprimé en grammes de I₂ par gramme d'ester, qui est inférieur à 10, de préférence inférieur à 5, encore mieux inférieur à 1, bien mieux encore inférieur à 0,5.

14. Utilisation selon l'une quelconque des revendications précédentes, dans laquelle au moins 90 % en masse du au moins un acide carboxylique sont des acides carboxyliques à chaîne linéaire, de préférence au moins 95 % en masse, encore mieux au moins 99 % en masse.

15. Concentré de pigment comprenant des particules d'un ou plusieurs pigments dans une quantité d'au moins 30 % en masse dispersés dans au moins un agent dispersant **caractérisé en ce que** le au moins un agent dispersant comprend un ester monomère d'un polyol avec au moins un groupe carboxylique selon la formule I 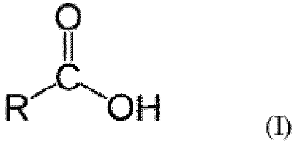 dans laquelle R est une moitié hydrocarbonée contenant de 3 jusqu'à 27 atomes de carbone, dans laquelle le polyol comprend de l'isosorbide et le degré moyen d'estérification des moitiés polyol dans l'ester d'isosorbide est de 1,50 à 1,99, de préférence de 1,60 à 1,98, en particulier de 1,70 à 1,97, encore mieux de 1,75 à 1,95 et dans laquelle l'ester monomère est un mélange comprenant des monoesters et polyesters d'isosorbide, lequel mélange comprend un monoester d'isosorbide à un niveau d'au plus 50 % en masse rapporté à la masse totale du mélange d'esters, de préférence d'au plus 30 % en masse de monoester, encore mieux d'au plus 20 % en masse et bien mieux encore d'au plus 10 % en masse de monoester.

16. Utilisation d'un concentré de pigment obtenu selon l'une quelconque des revendications 1-14 ou du concentré de pigment de la revendication 15 pour la préparation d'une composition d'encre d'impression offset.

17. Utilisation selon la revendication 16 pour la préparation d'une composition d'encre d'impression offset ou d'une composition de vernis offset utilisable avec un emballage alimentaire, de préférence avec un premier emballage alimentaire.

18. Utilisation d'un concentré de pigment obtenu selon l'une quelconque des revendications 1-14 ou du concentré de pigment de la revendication 15, pour améliorer la résistance de couleur et/ou la densité de couleur et/ou le brillant d'une composition d'encre d'impression offset ou d'une composition de vernis offset.

19. Utilisation d'un concentré de pigment obtenu selon l'une quelconque des revendications 1-14 ou du concentré de pigment de la revendication 15, pour la préparation d'une composition cosmétique, en particulier d'une crème, pommade, gel, lotion ou cire.
